# EUROPÄISCHE PATENTSCHRIFT

(11) **EP 1 199 295 B1**
(45) Veröffentlichungstag und Bekanntmachung des Hinweises auf die Patenterteilung: **17.09.2003**
(21) Anmeldenummer: 01124102.3
(22) Anmeldetag: 10.10.2001
(51) Int. Cl.: C07C 37/08

(54) **Verfahren zur Spaltung von Alkylarylhydroperoxiden**
Process for the cleavage of alkylaryl hydroperoxides
Procédé pour la scission des hydroperoxydes d'alkylaryles

(30) Priorität: 18.10.2000 DE 10051581
(43) Veröffentlichungstag der Anmeldung: 24.04.2002
(73) Patentinhaber: INEOS Phenol GmbH & Co. KG, 45966 Gladbeck (DE)
(72) Erfinder: Weber, Manfred, Dr., 45721 Haltern (DE); Tanger, Uwe, Dr., 44879 Bochum (DE)
(74) Vertreter: Sternagel, Fleischer, Godemeyer & Partner Patentanwälte

(56) Entgegenhaltungen:
- US-A- 6 057 483

## Beschreibung

Die Erfindung betrifft ein verbessertes Verfahren zur Spaltung von Alkylarylhydroperoxiden, insbesondere zur säurekatalysierten Spaltung von Cumolhydroperoxid (CHP) in Phenol und Aceton.

Das Verfahren der säurekatalysierten Spaltung von Cumolhydroperoxid in Phenol und Aceton ist seit langem von besonderer industrieller Bedeutung. Bei der Herstellung von Phenol aus Cumol nach dem Hock-Verfahren wird in einer ersten Reaktionsstufe, der so genannten Oxidation, Cumol zu Cumolhydroperoxid (CHP) oxidiert und das CHP anschließend in einer Vakuumdestillation, der so genannten Konzentrierung auf 65 bis 90 Gew.-% aufkonzentriert. In einer zweiten Reaktionsstufe, der so genannten Spaltung, wird das CHP durch Einwirken einer Säure, zumeist Schwefelsäure, in Phenol und Aceton gespalten. Dabei wird das bereits in der Oxidation gebildete Dimethylphenylcarbinol (DMPC) in einer Gleichgewichtsreaktion zum Teil in α-Methylstyrol (AMS) und Wasser gespalten, ein weiterer Teil des DMPCs reagiert mit CHP zu Dicumylperoxid (DCP), der Rest verbleibt in dem so genannten Spaltprodukt. Nach Neutralisation des Spaltproduktes wird dieses Produktgemisch üblicherweise destillativ aufgearbeitet.

Ein Teil des AMS oder des DMPCs bildet in der Spaltung Hochsieder (Dimere, Cumylphenole, Bisphenole), die in der Destillation als Rückstand ausgeschleust werden. Das nach der Neutralisation noch vorliegende AMS wird in der Destillation zu Cumol hydriert und zur Oxidation zurückgeführt. In der Spaltung nicht umgesetztes DMPC gelangt als Hochsieder in den Rückstand, zum Teil setzt es sich in den heißen Phenolkolonnen weiter um zu AMS, aus dem wiederum hochsiedende Nebenkomponenten entstehen. Das DCP ist bei üblichen Spalttemperaturen (50 bis 70 °C) stabil. Es zersetzt sich thermisch in den heißen Phenolkolonnen, wobei nach unseren Erfahrungen zum Teil o-Kresole gebildet werden. Unter Einfluss von Säure kann DCP bei Temperaturen oberhalb 80 °C dagegen in Phenol, Aceton und AMS gespalten werden. Es liegt daher nahe, unmittelbar nach der Spaltung das restliche DMPC und das in der Spaltung gebildete DCP vollständig umzusetzen, und zwar durch gezielte Erhöhung der Temperatur unter Einfluss der in der Spaltung als Katalysator verwendeten Säure. Dadurch wird DMPC weitgehend in AMS und DCP fast vollständig in Phenol, Aceton und ebenfalls AMS umgewandelt.

Eine derartige thermische Nachbehandlung des Spaltproduktes wurde bereits in US 2 757 209 beschrieben, wobei Temperaturen über 100°C, speziell von 110 bis 120°C verwendet wurden. Ziel dieser thermischen Nachbehandlung war die vollständige Dehydratisierung des DMPC zu AMS. In US 4 358 618 wird dagegen eine thermische Nachbehandlung beschrieben, die zum Ziel hat, das in der Spaltung gebildete DCP vollständig in Phenol, Aceton und AMS zu überführen, wobei Temperaturen von 120 und 150°C verwendet werden. In US 5 254 751 wird eine thermische Nachbehandlung mit gleicher Zielsetzung wie in US 4 358 618 beschrieben, wobei hier Temperaturen von 80 bis 110 °C verwendet werden. In DE 197 55 026 A1 schließlich wird die Nachbehandlung in einem Temperaturbereich von über 150 °C durchgeführt. Demnach gibt es in den bisherigen Beschreibungen drastische Unterschiede hinsichtlich optimaler Temperaturbereiche für die thermische Nachbehandlung von Spaltprodukt aus der Phenolherstellung.

In all diesen bisher beschriebenen Verfahren wird das Spaltprodukt für die thermische Nachbehandlung in Wärmeübertragern mit Dampf zunächst aufgeheizt und nach ausreichender Reaktionszeit in Wärmeübertragern mit Wasser wieder abgekühlt. Je nach gewählter Temperatur für die thermische Nachbehandlung ergeben sich dadurch spezifische Dampfverbräuche um 0,2 Tonnen Dampf je Tonne Phenol. Es wurde festgestellt, dass generell bei Temperaturen über 100°C, vor allem bei Temperaturen über 120°C ein verstärktes Ablagern von hochsiedenden Nebenprodukten in den Wärmeübertragern (Fouling) der thermischen Nachbehandlung, verbunden mit einem drastischen Rückgang des Wärmeübergangs auftritt. Insbesondere in den Apparaten zur Aufheizung des Produktes mit Dampf kommt es an den heißen Wärmeübertragungsflächen auf der Produktseite zur Ausbildung organischer Ablagerungen, so dass diese Apparate in relativ kurzen Zeitabständen von wenigen Wochen gereinigt werden müssen. Mit zunehmender Temperatur steigt dieses Fouling weiter an.

Durch Verwendung eines Rekuperators zur Aufheizung des Spaltproduktes, in dem das in die Nachtemperung eintretende Spaltprodukt durch das aus der Nachtemperung austretende Spaltprodukt vorgewärmt wird, lässt sich der Dampfverbrauch verringern. Das Problem des Auftretens von Foulingbildung wird durch diese Verwendung, wie sie in US 6 057 483 beschriebenen wird, nicht verhindert.

In DE 100 21 482 ist erstmals eine Lösung für diese Problematik vorgeschlagen worden. Dieses Verfahren zur thermischen Nachbehandlung von Spaltprodukt aus der säurekatalysierten Spaltung von Cumolhydroperoxid in Phenol und Aceton, wobei das thermisch zu behandelnde Spaltprodukt in einem Reaktor erwärmt wird, zeichnet sich dadurch aus, dass zum Erwärmen des thermisch zu behandelnden Spaltproduktes im Reaktor die Reaktionswärme zumindest einer in diesem Reaktor ablaufenden exothermen Reaktion genutzt wird. Auf diese Weise wird eine hohe Selektivität der Nachbehandlung bei gleichzeitiger Senkung der Energiekosten sowie eine höhere Standzeit der Wärmeübertrager durch Vermeiden von Fouling, erreicht.

Nachteilig an diesem Verfahren, bei dem üblicherweise die Reaktionswärme, die bei der Spaltung von CHP frei wird, genutzt wird, ist die Tatsache, dass zur Einstellung hoher Restgehalte an CHP am Ausgang der Spaltung und damit im Kreislaufstrom hohe Kreislaufströme erforderlich werden, um hiermit das in die Spaltung eintretende Konzentrat noch ausreichend verdünnen zu können. Eine zweite Zuspeisung von CHP-Konzentrat ist zwar vor diesem Hintergrund vorteilhafter, erfordert aber die für den Fachmann bekannten sicherheitstechnischen Einrichtungen und ist damit sehr aufwendig.

Damit stellt sich die Aufgabe, ein einfaches Verfahren zur Spaltung von Cumolhydroperoxid bereitzustellen, bei welchem auf die eigentliche thermische Nachbehandlung des Spaltprodukts aus der säurekatalysierten Spaltung von Cumolhydroperoxid in Phenol und Aceton, verzichtet werden kann, um eine hohe Sicherheit, geringe Investitionskosten sowie eine hohe Verfügbarkeit durch Vermeidung von Fouling zu erreichen.

Überraschenderweise wurde gefunden, dass durch ein Verfahren zur Spaltung von Alkylarylhydroperoxiden, wobei ein Gemisch aus einem zumindest ein zu spaltendes Alkylarylhydroperoxid aufweisendem Konzentrat und einem Spaltprodukt, welches aus der Spaltung eines Alkylarylhydroperoxid erhalten wird, hergestellt wird, dieses Gemisch in zumindest zwei Teilmengen aufgetrennt wird und die Alkylarylhydroperoxide in diesen Teilmengen parallel bei unterschiedlichen Temperaturen gespalten werden, sowohl eine hohe Selektivität der Spaltung bei gleichzeitiger Senkung der Energiekosten und eine höhere Standzeit der Wärmeübertrager durch Vermeiden von Fouling als auch eine höhere Sicherheit als bei herkömmlichen Verfahren erreicht wird.

Gegenstand der vorliegenden Erfindung ist deshalb ein Verfahren gemäß Anspruch 1 zur säurekatalysierten Spaltung von Alkylarylhydroperoxiden, wobei ein Gemisch aus einem zumindest ein zu spaltendes Alkylarylhydroperoxid aufweisendem Konzentrat und einem Spaltprodukt, welches aus der Spaltung eines Alkylarylhydroperoxid erhalten wird, hergestellt wird, dieses Gemisch in zumindest zwei Teilmengen aufgetrennt wird und die Alkylarylhydroperoxide in diesen Teilmengen parallel bei unterschiedlichen Temperaturen gespalten werden.

Das erfindungsgemäße Verfahren hat den Vorteil, dass gegenüber herkömmlichen Verfahren die thermische Nachbehandlung in der Spaltung integriert ist. Auf diese Weise wird wesentlich weniger Dampf zum Erwärmen des thermisch nachzubehandelnden Spaltproduktes benötigt. Bei einem genügend großen Betrag an frei werdender Reaktionswärme bei der Spaltung bei höherer Temperatur kann auf den Einsatz von Dampf zum Erwärmen des Spaltproduktes völlig verzichtet werden. Im Gegensatz zu Verfahren bzw. Vorrichtungen, bei welchen Dampf oder andere geeignete Wärmeträger dauernd zum Erwärmen des Spaltproduktes eingesetzt werden, tritt der Effekt des Foulings bei Einsatz des erfindungsgemäßen Verfahrens zur Behandlung des Spaltproduktes in wesentlich geringerem Ausmaß bzw. gar nicht auf. Im Gegensatz zu Verfahren, bei welchen zusätzliches Alkylarylhydroperoxid dem Spaltprodukt vor einer thermischen Nachbehandlung zugesetzt wird, benötigt das erfindungsgemäße Verfahren keine zusätzliche Einspeisestelle mit den dem Fachmann bekannten Sicherheitseinrichtungen.

Das erfindungsgemäße Verfahren eignet sich zur säurekatalysierten Spaltung von einem oder mehreren Alkyarylhydroperoxiden (AAHP), wie z.B. α-Methylbenzylhydroperoxid, α-Methyl-p-methylbenzylhydroperoxid, α,α-Dimethylbenzylhydroperoxid, auch bekannt als Isopropylbenzolhydroperoxid oder Cumolhydroperoxid (CHP), α,α-Methylethylbenzylhydroperoxid, auch bekannt als sek. Butylbenzolhydroperoxid, α,α-Dimethyl-p-methylbenzylhydroperoxid, α,α-Dimethyl-p-ethylbenzylhydroperoxid, a-Methyl-a-phenylbenzylhydroperoxid. Insbesondere eignet sich das erfindungsgemäße Verfahren zur säurekatalysierten Spaltung von Gemischen von Alkyarylhydroperoxiden, die zumindest Cumolhydroperoxid (CHP) aufweisen. Ganz besonders bevorzugt eignet sich das erfindungsgemäße Verfahren zur Spaltung von CHP.

Nachfolgend wird das erfindungsgemäße Verfahren beispielhaft anhand der säurekatalysierten Spaltung von CHP in Phenol und Aceton beschrieben, ohnc dass das erfindungsgemäße Verfahren auf diese Ausführungsart beschränkt sein soll.

Das erfindungsgemäße Verfahren zur Spaltung von Alkylarylhydroperoxiden, zeichnet sich dadurch aus, dass ein Gemisch aus einem zumindest ein zu spaltendes Alkylarylhydroperoxid aufweisendem Konzentrat und einem Spaltprodukt, welches aus der Spaltung zumindest eines Alkylarylhydroperoxids erhalten wird, hergestellt wird, dieses Gemisch in zumindest zwei Teilmengen aufgetrennt wird und die Alkylarylhydroperoxide in diesen Teilmengen des Gemisches parallel bei unterschiedlichen Temperaturen gespalten werden.

Die zumindest zwei Teilmengen des Gemisches werden vorzugsweise so behandelt, dass eine Teilmenge zur Spaltung von Alkylarylhydroperoxiden bei einer Temperatur von 45 bis 99 °C, vorzugsweise von 45 bis 90 °C behandelt wird und eine andere Teilmenge zur Spaltung von Alkylarylhydroperoxiden auf Temperaturen von über 100 °C erwärmt wird. Bei der Teilmenge, die auf Temperaturen über 100 °C erwärmt wird, findet eine Spaltung von Alkylarylhydroperoxiden mit integrierter thermischer Nachbehandlung statt. Im folgenden wird die Spaltung der Alkylarylhydroperoxide in der Teilmenge bei einer Temperatur von 45 bis 99 °C, vorzugsweise von 45 bis 90 °C als Niedertemperaturspaltung und die Spaltung der Alkylarylhydroperoxide in einer Teilmenge des Gemisches bei Temperaturen über 100 °C als Hochtemperaturspaltung bezeichnet.

Durch die Behandlung einer Teilmenge bei einer Temperatur von oberhalb 100 °C, also durch die Hochtemperaturspaltung werden Nebenprodukte, die bei einer Spaltung von Alkylarylhydroperoxiden oder in einem vorangegangenen Schritt des Gesamtverfahrens, wie z.B. der Oxidation entstanden sind, in verwertbare Produkte überführt. Im Fall der Herstellung von Phenol und Aceton nach dem Hock-Verfahren entsteht in der Oxidation z.B. Dimethylphenylcarbinol (DMPC) und während des Spaltvorganges aus DMPC und Cumolhydroperoxid (CHP) Dicumylperoxid (DCP). Das erfindungsgemäße Verfahren hat den Zweck, den Anteil an Dimethylphenylcarbinol (DMPC) und Dicumylperoxid (DCP) im Spaltprodukt, welches bei der Spaltung von CHP anfällt, zu verringern, da diese Verbindungen bei der anschließenden Aufarbeitung des Spaltproduktes, bei welcher üblicherweise mehrere destillative Schritte zur Stofftrennung durchgeführt werden, mit anderen Verbindungen oder mit sich selbst zu hochsiedenden, teerähnlichen Verbindungen weiterreagieren. Diese hochsiedenden Verbindungen können störend in den weiteren Prozessschritten zur Aufarbeitung von Spaltprodukt wirken. Durch die Bildung der Hochsieder wird außerdem die Ausbeute im Gesamtprozess der Hock'schen Phenolsynthese deutlich verringert.

Durch die Anwendung des erfindungsgemäßen Verfahrens auf die Spaltung von CHP werden das in dem zu spaltenden Gemisch enthaltende DMPC in α-Methylstyrol (AMS) und Wasser und das ebenfalls vorhandene DCP in Phenol, AMS und Aceton durch die Behandlung bei einer Temperatur von über 100 °C gespalten. Das bei diesen Reaktionen entstehende AMS lässt sich bei der weiteren Aufarbeitung des Spaltproduktes von diesem abtrennen und zu Cumol hydrieren, welches als Ausgangsstoff in den Gesamtprozess der Phenolherstellung rückgeführt werden kann. Auf diese Weise werden die Ausbeuteverluste durch Bildung von Nebenprodukten verringert.

Wie bereits beschrieben wird zumindest eine abgetrennte Teilmenge des Gemisches zur Spaltung zumindest eines Alkylarylhydroperoxids bei einer Temperatur von 45 bis 99 °C, vorzugsweise bei einer Temperatur von 45 bis 90 °C und ganz besonders bevorzugt bei einer Temperatur von 45 bis 75 °C behandelt. Diese Niedertemperaturspaltung kann in einem oder mehreren Spaltreaktoren durchgeführt werden, die eine genügend große Wärmeabfuhr aufweisen. Zur Einstellung der Temperatur kann es vorteilhaft sein, als Spaltreaktor z.B. einen Rohrbündelwärmeübertrager einzusetzen. Es kann aber auch jeder andere Reaktionsapparat verwendet werden, der geeignet ist, bei der Niedertemperaturspaltung frei werdende Reaktionswärme abzuführen. Vorzugsweise erfolgt die Niedertemperaturspaltung in zumindest zwei Spaltreaktoren, ganz besonders bevorzugt in zumindest drei Spaltreaktoren. Die Spaltreaktoren können parallel oder in Serie geschaltet sein. Vorzugsweise sind die Spaltreaktoren in Serie geschaltet und weisen zumindest eine Vorrichtung zum Abführen von Wärmeenergie auf.

Wird für die Behandlung der Teilmenge des Gemisches bei einer Temperatur von 45 bis 99 °C mehr als ein Spaltreaktor eingesetzt, so kann es vorteilhaft sein, wenn zumindest zwei der Spaltreaktoren einen Temperaturunterschied von 0 bis 10 °C, vorzugsweise von 2 bis 7 °C aufweisen. Vorzugsweise beträgt die Temperatur in zumindest einem der Spaltreaktoren von 45 bis 75 °C, in zumindest einem weiteren Spaltreaktor wird die Temperatur vorzugsweise so eingestellt, dass sie um 1 bis 10 °C, vorzugsweise um 2 bis 5 °C höher als im ersten Spaltreaktor ist, und, wenn vorhanden, beträgt die Temperatur in einem weiteren Spaltreaktor von 45 bis 75 °C.

Es kann vorteilhaft sein, wenn ein Teil oder die gesamte Menge des aus der Niedertemperaturspaltung erhaltenen Niedertemperaturspaltproduktes zur Erzeugung des Gemisches aus Spaltprodukt und Alkylarylhydroperoxid aufweisendem Konzentrat eingesetzt wird. Vorzugsweise wird die gesamte Menge des Niedertemperaturspaltproduktes zur Erzeugung des Gemisches eingesetzt.

Wie oben beschrieben wird zumindest eine andere Teilmenge des Gemisches bei einer Temperatur von über 100 °C behandelt. Vorzugsweise wird diese Teilmenge bei einer Temperatur von über 115 °C, besonders bevorzugt über 130 °C und ganz besonders bevorzugt über 150 °C behandelt. Vorzugsweise wird bei dieser Hochtemperaturspaltung die Teilmenge des Gemisches in einem Spaltreaktor durch eine in diesem Spaltreaktor ablaufenden exothermen Reaktion auf eine Temperatur von über 100 °C erwärmt. Es können unterschiedliche exotherme Reaktionen zum Erwärmen der Teilmenge eingesetzt werden. Vorzugsweise ist zumindest eine der im Spaltreaktor ablaufenden exothermen Reaktionen die Spaltung eines Alkylarylhydroperoxids. Handelt es sich bei dem zur Erzeugung des Gemisches eingesetzten Konzentrat um ein Konzentrat, welches bei der Hock'schen Phenolsynthese erhalten wird, so ist zumindest eine der exothermen Reaktionen die Spaltung von Cumolhydroperoxid.

Für den Fall der Spaltung von CHP wurde gefunden, dass bei Temperaturen über 100 °C auch bei noch nicht vollständigem Umsatz an DCP die Umwandlung von DMPC in AMS und Wasser entsprechend dem thermodynamischen Gleichgewicht bereits zum großen Teil erfolgt ist. Somit muss zur Einstellung optimaler Bedingungen bei Fahrweisen oberhalb 100 °C allein der DCP-Restgehalt nach der thermischen Behandlung der Teilmenge des Gemisches überprüft werden. Vorzugsweise beträgt der Restgehalt an DCP in der erfindungsgemäß bei einer Temperatur von über 100 °C thermisch behandelten Teilmenge des Gemisches von 0,001 bis 1 Gew.-%, vorzugsweise von 0,01 bis 0,1 Gew.-%. Höhere Werte führen zu einer Verschlechterung der Selektivität des Gesamtprozesses eben über diese DCP-Verluste, die darüber hinaus zu höheren Gehalten an o-Kresol im Reinphenol führen können, niedrige Werte von unter 0,01 Gew.-% können zu einer zu hohen Nebenproduktbildung von Hochsiedern aus AMS oder DMPC während der Behandlung der Teilmenge des Gemisches bei einer Temperatur über 100 °C führen. Üblicherweise wird der DCP-Restgehalt analytisch ermittelt. Aus den genannten Gründen wird bei der Hochtemperaturspaltung die thermisch zu behandelnde Teilmenge des Gemisches auf eine Temperatur von über 100 °C, vorzugsweise von über 115 °C, besonders bevorzugt auf eine Temperatur von über 130 °C und ganz besonders bevorzugt auf eine Temperatur von über 150 °C erwärmt.

Zur Hochtemperaturspaltung der Alkylarylhydroperoxide in der Teilmenge des Gemisches wird diese in einen Reaktor, vorzugsweise in einen Reaktor, der die Charakteristik eines Rohrreaktors aufweist, überführt und auf über 100 °C erwärmt. Erfindungsgemäß erfolgt das Erwärmen bzw. Erhitzen dieser Teilmenge durch Nutzen der Reaktionswärme, die beim Ablauf zumindest einer exothermen Reaktion in der Teilmenge entsteht. Erfindungsgemäß ist eine der exothermen Reaktionen die säurekatalysierte Spaltung von CHP. Da das Erwärmen der Teilmenge des Gemisches durch Nutzung der Reaktionswärme zumindest einer exothermen Reaktion direkt erfolgt, kann auf eine indirekte Wärmeübertragung mittels Wärmeübertragern zur Erwärmung der Teilmenge des Gemisches ganz verzichtet werden.

Auch durch die Spaltung von DCP über CHP und DMPC in Phenol, Aceton und AMS wird, da es sich ebenfalls um exotherme Reaktionen handelt, ebenfalls Reaktionswärme frei, die einer definierten Erhöhung der Temperatur in der Teilmenge des Gemisches entspricht. Diese Temperaturdifferenz liegt je nach Anfangsgehalt an DCP üblicherweise zwischen 10 und 20 °C. Typische Konzentrationen von DCP liegen im Bereich von 2 bis 8 Gew.-%. Das erfindungsgemäße Verfahren soll aber nicht auf die angegebenen Konzentrationen für DCP beschränkt sein.

Die durch diese oben genannten exothermen Reaktionen frei werdende Wärmemenge muss bei der Berechnung der erforderlichen Anfangskonzentration von CHP in der Teilmenge des Gemisches, die auf eine Temperatur von über 100 °C erwärmt werden soll, berücksichtigt werden.

Zum Erreichen der gewünschten Temperaturen von über 100 °C, vorzugsweise von über 115 °C, besonders bevorzugt von über 130 °C und ganz besonders bevorzugt von über 150 °C weist die thermisch zu behandelnde Teilmenge des Gemisches vor der thermischen Behandlung vorzugsweise eine Cumolhydroperoxid-Konzentration von 2 bis 25 Gew.-% auf.

Vorzugsweise weist die Teilmenge des Gemisches eine CHP-Konzentration von 5 bis 20 Gew.-%, ganz besonders bevorzugt von 10 bis 15 Gew.-%, auf. Die notwendige Konzentration an CHP in der Teilmenge ist abhängig von der Starttemperatur der Teilmenge des Gemisches und von der DCP-Ausgangskonzentration. Zur Berechnung der notwendigen CHP-Konzentration kann die Faustformel herangezogen werden, dass die Spaltung von einer 1 Gew.-%-igen CHP-Lösung ungefähr so viel Wärme freisetzt, wie zur Erhöhung der Temperatur der Lösung um 6,8 bis 7,0 °C notwendig ist. Eine 6 Gew.-%-ige CHP-Lösung würde also durch Spaltung des gesamten CHPs um 40,8 bis 42 °C erwärmt werden. Die Faustformel gilt für die bei der CHP-Spaltung üblicherweise eingesetzten Lösungen. Diese weisen üblicherweise zumindest Cumol, Phenol und Aceton aber nur geringe Mengen (von 0 bis 15 Gew.-%) an Wasser auf. Auf Grund der höheren Wärmekapazität des Wassers würde die Spaltung von CHP in einer Lösung bzw. Dispersion, die 99 Gew.-% Wasser und 1 Gew.-% CHP aufweist diese Lösung nur um 3,5 °C erhöhen. Für Teilmengen, die einen höheren Wasseranteil als üblich aufweisen, muss deshalb der Erwärmungsfaktor neu bestimmt werden. Diese Bestimmung kann auf eine dem Fachmann bekannte Weise in einfachen Vorversuchen erfolgen.

Nach der oben genannten Faustformel beträgt z.B. bei einer Starttemperatur einer Teilmenge eines Gemisches, direkt nach der Trennung des Gemisches, von 40 °C und einem DCP-Gehalt von 4 Gew.-% die erforderliche CHP-Konzentration in der Teilmenge ca. 8,5 Gew.-% um auf eine End- bzw. Behandlungstemperatur bei der Hochtemperaturspaltung von 115 °C zu kommen. Um auf eine Endtemperatur bei der Hochtemperaturspaltung von 175 °C zu kommen, müsste der Gehalt an CHP in der Teilmenge des Gemisches ca.20 Gew.-% betragen.

Die Aufheizzeit der Teilmenge des Gemisches auf eine Temperatur bis 100 °C bei der Hochtemperaturspaltung beträgt nach der Trennung des Gemisches üblicherweise weniger als 30 sec. In der anschließenden eigentlichen Hochtemperaturspaltung steigt die Temperatur des Gemisches im Verweilzeitreaktor auf eine Temperatur von über 100 °C an. Die Verweilzeit des Spaltproduktgemisches im Verweilzeitreaktor ist abhängig von der Säurestärke. Je nach Säurestärke beträgt die Verweilzeit üblicherweise von 5 bis 600 sec.

In einer besonders bevorzugten Ausführungsart des erfindungsgemäßen Verfahrens werden die genannten Information bzw. Faustformeln zur Steuerung der Spaltung von Cumolhydroperoxid verwendet. Aus dem Temperaturprofil in dem Hochtemperaturspaltreaktor, welches im Wesentlichen durch die exotherme Reaktion der Cumolhydroperoxidspaltung bzw. der bei dieser Reaktion frei werdenden Wärmemenge beeinflusst wird, kann die Ausgangskonzentration an CHP in dem Gemisch vor der Trennung des Gemisches in zwei Teilmengen errechnet werden. Durch die Verwendung des Hochtemperaturspaltreaktors als Kalorimeter lassen sich Informationen über die Zusammensetzung des Gemisches gewinnen, welche zur Steuerung der Spaltung, insbesondere der Hochtemperaturspaltung genutzt werden kann.

Es kann aber auch vorteilhaft sein, den Reaktor, der die Charakteristik eines Rohrreaktors aufweist und in welchem die Hochtemperaturspaltung von Alkylarylhydroperoxiden in der Teilmenge des Gemisches bei einer Temperatur von über 100 °C stattfindet, mit einer Möglichkeit zum Abführen von Wärmeenergie auszurüsten. Auf diese Weise kann die Temperatur, bei welcher die Hochtemperaturspaltung der Alkylarylhydroperoxide stattfindet, auch in dem Fall nach oben hin begrenzt werden, in dem der Produktstrom eine Zusammensetzung aufweist, bei welcher die Summe der bei den exothermen Reaktionen frei werdenden Wärmemengen größer ist, als zur Aufheizung bzw. Erwärmung der Teilmenge des Gemisches benötigt wird. Die bevorzugte maximale Temperatur für die Hochtemperaturspaltung der Alkylarylhydroperoxide in dieser Teilmenge des Gemisches, liegt im Fall der Spaltung von CHP bei unter 200 °C. Beim Überschreiten dieser Temperatur in der thermischen Spaltung lässt sich eine vermehrte Bildung von Nebenprodukten durch thermische Zersetzung feststellen, welche mit einer Verringerung der Ausbeute an Phenol und/oder AMS in der Spaltung einhergeht.

Nach der Hochtemperaturspaltung der Alkylarylhydroperoxide in dieser Teilmenge des Gemisches durch Behandlung der Teilmenge bei einer Temperatur über 100 °C kann das dabei erhaltene Hochtemperaturspaltprodukt in einem Kühler abgekühlt werden. Vorzugsweise wird das Hochtemperaturspaltprodukt auf eine Endtemperatur von üblicherweise 40 bis 70 °C gebracht. Es kann vorteilhaft sein, vor dem Reduzieren der Temperatur des durch Behandlung bei einer Temperatur von über 100 °C erhaltenen Hochtemperaturspaltproduktes dieses mit zumindest einer Base, zu neutralisieren. Als Basen können organische oder anorganische Basen eingesetzt werden. Vorzugsweise wird Natronlau (NaOH) und/oder Phenolatlauge (NaOPh) als Base eingesetzt. Die Base kann erwäri oder kalt, das heißt mit einer Temperatur, die der Umgebungstemperatur entspricht, de Hochtemperaturspaltprodukt zugegeben werden. Vorzugsweise wird die Base kalt de 5 Hochtemperaturspaltprodukt zugegeben. Die Zugabe kann durch Zumischen der Base zu Hochtemperaturspaltprodukt aus der Behandlung bei einer Temperatur von über 100° mittels geeigneter Apparaturen, wie z.B. einem oder mehreren statischen Mischern, erfolge Durch die Zugabe von Base vor dem Abkühlen des Hochtemperaturspaltproduktes kar erreicht werden, dass säurekatalysierte Reaktionen direkt nach Zugabe der Base gestop werden können, wodurch insbesondere unerwünschte säurekatalysierte Nebenreaktionen, d während des Abkühlens und bis zur üblicherweise nach dem Abkühlen stattfindende Neutralisation auftreten können, gestoppt werden. Die Base kann als wässrige Lösun zugegeben werden. Es kann vorteilhaft sein, wenn durch Zugabe der Base in wässrige Lösung so viel Wasser dem Hochtemperaturspaltprodukt aus der Behandlung bei eine 5 Temperatur von über 100 °C zugeführt wird, dass der Wassergehalt in diesem Spaltprodul oberhalb oder unterhalb der Sättigungskonzentration für Wasser liegt oder der Wassergeha der Sättigungskonzentration für Wasser entspricht.

Das erfindungsgemäß erhaltene Hochtemperaturspaltprodukt aus der Behandlung bei eine Temperatur von über 100 °C wird einer Weiterbehandlung oder Aufarbeitung zugeführ Üblicherweise wird dieses Hochtemperaturspaltprodukt aus der CHP-Spaltung so aufge arbeitet, dass Aceton und Phenol destillativ voneinander und von weiteren im erfindungs gemäß behandelten Hochtemperaturspaltprodukt vorhandenen Verbindungen getrennt werden. Die Aufarbeitung dieser Spaltproduktströme ist dem Fachmann bekannt.

Es kann vorteilhaft sein, wenn der thermisch bei einer Temperatur von über 100 °C z behandelnden Teilmenge des Gemisches vor dem Eintritt dieser Teilmenge in den Reaktor, i: dem die Hochtemperaturspaltung stattfinden soll, Wasser zudosiert wird. Besonder bevorzugt wird der Teilmenge vor der Hochtemperaturspaltung so viel Wasser zugesetzt, das die Konzentration von Wasser in dieser Teilmenge des Gemisches von 0,1 bis 3,0 Gew.-% vorzugsweise von 0,5 bis 2 Gew.-% und ganz besonders bevorzugt von 0,5 bis 1,0 Gew.-% beträgt.

Durch das erfindungsgemäße Verfahren zur Spaltung von Alkylarylhydroperoxiden, kann bei der Spaltung von CHP mit einer Konzentration von 65 bis 90 Gew.-% im CHP-Konzentrat ein Hochtemperaturspaltprodukt aus der bei einer Temperatur von über 100 °C behandelten Teilmenge erhalten werden, das einen Restgehalt an Dicumylperoxid in von 0,001 bis 1 Gew.-%, ganz besonders bevorzugt von 0,01 bis 0,1 Gew.-% aufweist. Durch Anwendung des erfindungsgemäßen Verfahrens bei der Spaltung von CHP weist das Hochtemperaturspaltprodukt eine Konzentration an DMPC von 0,05 bis 0,2 Gew.-% auf. CHP ist in diesem Hochtemperaturspaltprodukt nicht mehr nachweisbar.

Das Gemisch aus zumindest ein zu spaltendes Alkylarylhydroperoxid aufweisendem Konzentrat und einem Spaltprodukt wird vorzugsweise durch Mischen des Konzentrat mit dem Spaltprodukt im Verhältnis von 1: 100 bis 1 : 1, besonders bevorzugt von 1 : 50 bis 1 : 5 erzeugt.

Vorzugsweise weist das zum Erzeugen des Gemisches verwendete zumindest ein Alkylarylhydroperoxid (AAHP) aufweisende Konzentrat von 40 bis 95 Gew.-% zumindest eines Alkylarylhydroperoxides, besonders bevorzugt von 50 bis 90 Gew.-% eines Alkylarylhydroperoxides und im Fall von CHP besonders bevorzugt von 65 bis 90 Gew. -% von zumindest CHP auf. Weitere Bestandteile des Konzentrats können im Falle des CHPs unter anderem Cumol, DMPC, DCP, Acetophenon, AMS und/oder Wasser sein.

Das Mischen erfolgt vorzugsweise so, dass eine ausreichende Durchmischung des Alkylarylhydroperoxide aufweisenden Konzentrats mit dem Spaltprodukt erreicht wird. Dies kann auf eine dem Fachmann bekannte Weise, z.B. durch Einbauten, die eine vollständige Durchmischung ermöglichen, wie z.B. statische Mischer, sichergestellt werden. Es ist auch möglich die Zudosierung des Alkylarylhydroperoxide aufweisenden Konzentrats zum Spaltprodukt auf der Saugseite der Pumpe, welche das Spaltprodukt pumpt, vorzunehmen. Auch auf diese Weise wird das Erreichen einer vollständigen Durchmischung des Spaltproduktes mit dem zudosierten Konzentrat sichergestellt. Eine ausreichende Durchmischung des Konzentrats mit dem Spaltprodukt ist notwendig, um eine lokale Überhitzung des Gemisches zu vermeiden.

Das mit dem Alkylarylhydroperoxide aufweisende Konzentrat zu mischende Spaltprodukt weist vor der Mischung mit dem Konzentrat vorzugsweise eine Konzentration an Alkylarylhydroperoxiden von 0 bis 20 Gew.-%, vorzugsweise von 1 bis 10 Gew.-% auf.

Es kann vorteilhaft sein, wenn das Spaltprodukt, welches mit dem Konzentrat gemischt wird, eine äquimolare oder nahezu äquimolare Menge an den Hauptfolgeprodukten der Spaltreaktion, also einem Keton und einem zumindest eine OH-Gruppe aufweisenden Aromaten, aufweist. Im Fall der Spaltung von CHP weist das zur Erzeugung des Gemisches verwendete Spaltprodukt vorzugsweise eine äquimolare Menge an Aceton und Phenol auf.

Es kann aber ebenso vorteilhaft sein, wenn das Spaltprodukt, mit welchem das Konzentrat zur Erzeugung des Gemisches gemischt wird, bei der Spaltung von CHP ein molares Verhältnis von Aceton zu Phenol 1,1 : 1 bis 4 : 1, vorzugsweise von 1,2 : 1 bis 1,5 : 1 aufweist. Dies kann z.B. dadurch erreicht werden, dass ein Teil eines bei einer späteren destillativen Aufarbeitung aus dem Hochtemperaturspaltprodukt gewonnen Acetons als Verdünnungsmittel in das Niedertemperaturspaltprodukt zurückgefahren wird. Eine solche Acetonrückführung ist für ein anderes Verfahren zur Spaltung von AAHP's in ähnlicher Weise z.B. in US 5 254 751 beschrieben.

In einer ganz besonders bevorzugten Ausführungsart des erfindungsgemäßen Verfahrens wird als Spaltprodukt, welches aus der Spaltung eines Alkylarylhydroperoxid erhalten wird und welches zum Erzeugen des Gemisches aus Spaltprodukt und Konzentrat verwendet wird, das Niedertemperaturspaltprodukt aus der Niedertemperaturspaltung zumindest einer der Teilmengen des Gemisches eingesetzt wird. Vorzugsweise wird als Spaltprodukt das Niedertemperaturspaltprodukt einer Teilmenge des Gemisches, welche bei einer Temperatur von 45 bis 90 °C behandelt wurde, eingesetzt. Ganz besonders bevorzugt wird das gesamte Spaltprodukt aus der Niedertemperaturspaltung zum Mischen mit dem Konzentrat eingesetzt.

Bei der Verwendung des gesamten Niedertemperaturspaltproduktes zum Mischen des Gemisches aus Spaltprodukt und Konzentrat wird das erfindungsgemäße Verfahren so betrieben, dass die Spaltung der Alkylarylhydroperoxide in zumindest zwei Stufen,
a) der säurekatalysierten Niedertemperaturspaltung von Alkylarylhydroperoxiden in einem oder mehreren Spaltreaktoren, wobei das Niedertemperaturspaltprodukt zur Erzeugung eines Gemisches mit einem zumindest ein Alkylarylhydroperoxid aufweisendem Konzentrat gemischt wird, und
b) Durchführung einer Hochtemperaturspaltung mit einer Teilmenge des Gemisches aus Spaltprodukt aus Schritt a und einem Alkylarylhydroperoxid aufweisendem Konzentrat, wobei die Teilmenge des Gemisches in einem Reaktor erwärmt wird und zum Erwärmen dieser Teilmenge im Reaktor die Reaktionswärme zumindest einer in diesem Reaktor ablaufenden exothermen Reaktion genutzt wird, durchgeführt wird.

Das erfindungsgemäße Verfahren zeichnet sich dadurch aus, dass die Konzentrationen an Alkylarylhydroperoxiden in den Teilmengen des Gemisches direkt vor und damit auch direkt nach dem Auftrennen des Gemisches gleich sind. Bei der Anwendung des erfindungsgemäßen Verfahren in der Phenol-Synthese nach dem Hock-Verfahren weisen die Gemische als Alkylarylhydroperoxid vorzugsweise zumindest Cumolhydroperoxid auf.

Die Teilmengen des Gemisches weisen vorzugsweise direkt nach dem Auftrennen des Gemisches Alkylarylhydroperoxide, vorzugsweise zumindest Cumolhydroperoxid in einer Konzentration von bis zu 25 Gew.-%, besonders bevorzugt von 5 bis 20 Gew.-% und ganz besonders bevorzugt von 10 bis 15 Gew.-% auf.

Das Auftrennen des Gemisches in zumindest zwei Teilmengen kann während der Herstellung des Gemisches, nach der Herstellung des Gemisches aber vor einer ersten Behandlung des Gemisches zur Spaltung von zumindest einem Alkylarylhydroperoxid in einem Reaktor oder nach der Herstellung des Gemisches und während, aber zumindest vor Beendigung, zumindest einer Behandlung des Gemisches zur Spaltung von zumindest einem Alkylarylhydroperoxid in zumindest einem Reaktor erfolgen.

Vorzugsweise wird die Trennung des Gemisches nach der Einspeisung des zu spaltenden Alkylarylhydroperoxids zum Spaltprodukt und vor Eintritt dieses Gemisches in einen ersten Spaltreaktor vorgenommen. Insbesondere kann es vorteilhaft sein, wenn die Trennung des Gemisches direkt nach der Einspeisung des zu spaltenden Alkylarylhydroperoxids zum Spaltprodukt erfolgt.

Je nach Reaktivität der als Konzentrat zugefügten Alkylarylhydroperoxide kann es aber auch vorteilhaft sein, dass die Trennung des Gemisches nach der Einspeisung des zu spaltenden Alkylarylhydroperoxids zum Spaltprodukt, nach Eintritt dieses Gemisches in zumindest einen Spaltreaktor, nach Austritt dieses Spaltgemisches aus zumindest einem Spaltreaktor aber zumindest bevor das Gemisch als Spaltprodukt einen letzten Spaltreaktor verlässt, erfolgt. Je nach der Anzahl der eingesetzten Spaltreaktoren kann das Gemisch an unterschiedlichen Stellen getrennt werden. So kann das Gemisch bei der Verwendung von drei in Serie geschalteten Spaltreaktoren, vor Eintritt des Gemisches in den ersten Spaltreaktor, nach Austritt aus dem ersten oder nach Austritt aus dem zweiten Spaltreaktor getrennt werden. Auch eine prinzipiell mögliche Ausschleusung innerhalb der Spaltreaktoren fällt unter die Ansprüche des erfindungsgemäßen Verfahrens. Im Folgenden wird die Durchführung einer ersten Spaltung von Alkylarylhydroperoxiden in zumindest einem Spaltreaktor vor der Trennung des Gemisches als Vorspaltung bezeichnet.

Erfolgt die Trennung während, aber zumindest vor Beendigung, zumindest einer Behandlung des Gemisches zur Spaltung von zumindest einem Alkylarylhydroperoxid in zumindest einem Reaktor, wird das erfindungsgemäße Verfahren also so betrieben, dass eine Vorspaltung durchgeführt wird, so wird das Gemisch zur Spaltung zumindest eines Alkylarylhydroperoxids in zumindest einem Spaltreaktor bei 45 bis 99 °C, vorzugsweise bei 45 bis 90 °C und ganz besonders bevorzugt bei 45 bis 75 °C behandelt. Vorzugsweise wird diese Vorspaltung in zumindest einem Reaktor, der mit einer Vorrichtung zur Abfuhr von Wärmeenergie, wie z.B. einem Wärmetauscher ausgestattet ist, durchgeführt.

Da zur thermischen Behandlung einer Teilmenge des Gemisches bei über 100 °C die thermisch zu behandelnde Teilmenge in einem Reaktor erwärmt wird und zum Erwärmen der thermisch zu behandelnden Teilmenge im Reaktor die Reaktionswärme zumindest einer in diesem Reaktor ablaufenden exothermen Reaktion genutzt wird und vorzugsweise zumindest eine dieser exothermen Reaktionen die Spaltung eines Alkylarylhydroperoxids ist, kann durch die Wahl der entsprechenden Stelle, an der das Trennen des Gemisches erfolgen soll, erreicht werden, dass die Konzentration an Alkylarylhydroperoxid in den Teilmengen, insbesondere in der Teilmenge des Gemisches, die auf eine Temperatur von über 100 °C erwärmt werden soll, gerade so groß ist, dass die bei den in diesem Reaktor ablaufenden exothermen Reaktionen frei werdende Wärmeenergie gerade so groß ist, dass die gewünschte Temperatur im Reaktor erreicht wird.

Je nach Konzentration und Menge des zum Spaltprodukt zudosierten zumindest ein Alkylarylhydroperoxid aufweisenden Konzentrats, sowie der Wärmeenergie, die bei der exothermen Reaktion frei wird, erfolgt das Auftrennen des Gemisches in zumindest zwei Teilmengen nach einer Reaktionszeit von 0,01 bis 600, vorzugsweise 0,1 bis 120 Sekunden nach Erzeugung der Gemisches aus zu spaltendes Alkylarylhydroperoxid aufweisendem Konzentrat und Spaltprodukt.

Das Verhältnis von Menge der Teilmenge, die der Niedertemperaturspaltung zugeführt wird, zur Menge der Teilmenge, die der Hochtemperaturspaltung zugeführt wird, entspricht, wenn das gesamte Niedertemperaturspaltprodukt zur Erzeugung des Gemisches verwendet wird, dem Mischungsverhältnis von der Menge Spaltprodukt zur Menge Alkylarylhydroperoxid aufweisendem Konzentrat, wie es zur Erzeugung des Gemisches eingesetzt wird.

Das erfindungsgemäße Verfahren kann zur Herstellung von Phenol durch Spaltung von Cumolhydroperoxid, wobei ein Gemisch aus einem zumindest ein zu spaltendes Alkylarylhydroperoxid aufweisendem Konzentrat und einem Spaltprodukt, welches aus der Spaltung eines Alkylarylhydroperoxid erhalten wird, hergestellt wird, dieses Gemisch in zumindest zwei Teilmengen aufgetrennt wird und die Alkylarylhydroperoxide in diesen Teilmengen parallel bei unterschiedlichen Temperaturen gespalten werden, eingesetzt werden.

Durch das erfindungsgemäße Verfahren ist ein Gemisch erhältlich, welches Phenol, Aceton, AMS und Cumol aufweist.

Das erfindungsgemäße Verfahren kann, wie bereits oben angesprochen, bei allen Verfahren eingesetzt werden, in denen Alkylarylhydroperoxide gespalten werden. Alkylarylhydroperoxide können z.B. Cumolhydroperoxid, sek-Butylbenzolhydroperoxid aber auch substituierte Alkylbenzolhydroperoxide oder Alkylhydroperoxide anderer Aromaten, wie z. B. Naphthalin, sein. Vorzugsweise wird das erfindungsgemäße Verfahren bei der Spaltung von Alkylarylhydroperoxiden eingesetzt, bei denen die Spaltung eine exotherme Reaktion ist. Es ist aber auch möglich das erfindungsgemäße Verfahren zur Spaltung von Konzentraten, die mehr als ein Alkylarylhydroperoxid aufweisen, einzusetzen. In diesem Fall muss zumindest eine der Spaltreaktionen eine exotherme Reaktion sein. Ganz besonders bevorzugt wird das erfindungsgemäße Verfahren bei der säurekatalysierten Spaltung von CHP in Phenol und Aceton mit kombinierter thermischer Nachbehandlung des Spaltproduktes eingesetzt.

Das erfindungsgemäße Verfahren kann kontinuierlich oder diskontinuierlich durchgeführt werden. Vorzugsweise wird das erfindungsgemäße Verfahren kontinuierlich durchgeführt.

Das erfindungsgemäße Verfahren kann so durchgeführt werden, dass die säurekatalysierte Spaltung von Alkylarylhydroperoxiden in homogener Phase oder in heterogener Phase durchgeführt wird. Wird die Niedertemperaturspaltung in heterogener Phase durchgeführt ist es notwendig, dass auch bei der Hochtemperaturspaltung ein heterogener Katalysator vorhanden ist. Wird das Verfahren so durchgeführt, dass eine Vorspaltung stattfindet, so muss auch bei der Vorspaltung ein heterogener Katalysator vorhanden sein. Als in heterogener oder homogener Phase einzusetzende Katalysatoren können alle im Stand der Technik beschriebenen Katalysatoren verwendet werden. Vorzugsweise werden die Spaltungen in dem erfindungsgemäßen Verfahren, also die Vor-, Niedertemperatur- und die Hochtemperaturspaltung, in homogener Phase durchgeführt.

Vorzugsweise wird zur Spaltung von Alkylarylhydroperoxid bzw. von CHP Schwefelsäure als Katalysator eingesetzt. Vorzugsweise weist das Gemisch eine Konzentration an Schwefelsäure von 50 bis 1 000 wppm auf. Die Zugabe des Katalysators erfolgt vorzugsweise in das zur Erzeugung des Gemisches verwendete Spaltprodukt.

Es kann vorteilhaft sein, die Säureaktivität, dass heißt die Säurestärke der Teilmenge des Gemisches, die der Hochtemperaturspaltung zugeführt wird, zu verändern. Die Säurestärke ist abhängig von der Säurekonzentration und der Konzentration an Wasser im Spaltgemisch. Je höher der Wassergehalt im Gemisch ist, desto mehr Säure muss dem Gemisch zudosiert werden, um auf dic gleiche Säureaktivität zu kommen, wobei die Wasserkonzentration bei der Berechnung der Säurestärke zum Quadrat eingeht. So beträgt zum Beispiel die Säurestärke einer Spaltgemisch-Lösung, die 200 wppm Schwefelsäure und 2 Gew.-% Wasser nur ca. ein Sechzehntel der Säurestärke einer Spaltgemisch-Lösung die 200 wppm Schwefelsäure und 0,5 Gew.-% Wasser aufweist.

Die ideale Säurestärke und damit die ideale Zusammensetzung des Gemisches, bzw. der Teilmengen des Gemisches in Bezug auf Säurekonzentration und Wasserkonzentration kann durch einfache Vorversuche ermittelt werden. Bei Gemischen, die eine Wasserkonzentration von typischerweise bis zu 2 Gew.-% aufweisen, erweist sich eine Schwefelsäurekonzentration von 100 bis 500 wppm im Gemisch als besonders vorteilhaft. Zur Erhöhung der Säurestärke wird üblicherweise Schwefelsäure nachdosiert. Zur Senkung der Säurestärke kann dem Gemisch eine Base, wie z. B. Phenolatlauge, Ammoniak oder Natronlauge, oder Wasser hinzugefügt werden. Vorzugsweise wird dem Gemisch Wasser hinzugefügt.

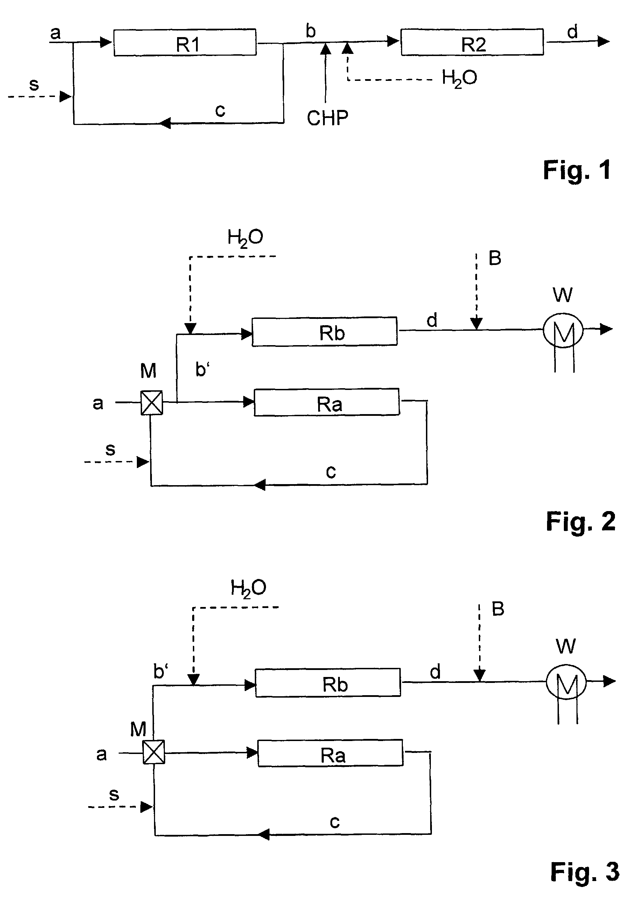

Eine Vorrichtung bzw. ein Verfahren nach dem Stand der Technik wird in Fig. 1 dargestellt. Unterschiedliche Ausführungsformen des erfindungsgemäßen Verfahrens bzw. der erfindungsgemäßen Vorrichtung werden in den Abbildungen Fig. 2 bis Fig. 5 beispielhaft beschrieben, ohne dass das Verfahren bzw. die Vorrichtung auf diese Ausführungsarten beschränkt sein soll.

In Fig. 1 ist schematisch ein Verfahren zur Spaltung von CHP gemäß dem Stand der Technik (DE 100 21 482) dargestellt. Über Leitung **a** wird ein Konzentrat, welches das zu spaltende CHP aufweist in einen ersten Reaktor, den Spaltreaktor, eingespeist. Bei dem Spaltreaktor **R1** muss es sich nicht um nur einen Reaktor handeln, der z.B. als Rohrreaktor mit Rückführung oder als rückvermischter Apparat ausgeführt sein kann, es können auch mehrere hintereinander geschaltete Reaktoren als Spaltreaktor bezeichnet werden. Das aus dem Spaltreaktor austretende Spaltprodukt wird im Falle eines Rohrreaktors zumindest teilweise über Leitung c im Kreis zurück in den Spaltreaktor gefahren. Über Leitung s kann, bei Einsatz eines homogenen Katalysators, dieser in das Spaltprodukt gegeben werden. Über Leitung **b** wird ein Teil des Spaltproduktgemisches in einen zweiten Reaktor **R2** gefahren, in welchem die thermische Nachbehandlung stattfindet. Vor dem Reaktor **R2** kann über zwei Leitungen zusätzlich Cumolhydroperoxid (**CHP**) und optional Wasser (**H**_{**2**}**O**) in das Spaltproduktgemisch gefahren werden. Über Leitung **d** verlässt das thermisch nachbehandelte Spaltproduktgemisch den Reaktor **R2** und kann einer Aufarbeitung zugeführt werden.

In Fig. 2 ist schematisch ein erfindungsgemäßes Verfahren zur Spaltung von CHP dargestellt. Über Leitung **a** wird ein Konzentrat, welches das zu spaltende CHP aufweist über einen Mischer **M** mit dem Spaltprodukt **c**, aus dem Reaktor **R**a zu einem Gemisch vermischt. Über Leitung **s** kann, bei Einsatz eines homogenen Katalysators, dieser in das Spaltprodukt **c** gegeben werden. Eine Teilmenge des Gemisches aus dem Mischer **M** wird in einen ersten Reaktor **Ra**, den Niedertemperaturspaltreaktor, eingespeist. Bei dem Niedertemperaturspaltreaktor **Ra** kann es sich nicht nur um einen Reaktor handeln, der z. B. als Rohrreaktor mit Rückführung oder als rückvermischter Apparat ausgeführt sein kann, es können auch mehrere hintereinander geschaltete Reaktoren als Niedertemperaturspaltreaktor bezeichnet werden. Das aus dem Niedertemperaturspaltreaktor austretende Niedertemperaturspaltprodukt wird im Falle eines Rohrreaktors über Leitung **c** über den Mischer **M** im Kreis zurück in den Niedertemperaturspaltreaktor gefahren. Über Leitung b' wird ein Teil des Gemisches vor Eintritt in den Reaktor **Ra** in einen zweiten Spaltreaktor **Rb**, den Hochtemperaturspaltreaktor gefahren, in welchem die thermische Hochtemperaturspaltung stattfindet. Vor dem Reaktor **Rb** kann über eine Leitung optional Wasser (**H**_{**2**}**O**) in die Teilmenge des Gemisches gefahren werden. Über Leitung **d** verlässt das Hochtemperaturspaltprodukt den Reaktor **R**b und kann einer Aufarbeitung zugeführt werden. Mittels des Wärmetauschers **W** kann dem Hochtemperaturspaltprodukt Wärme entzogen werden. Optional kann dem Hochtemperaturspaltprodukt vor Eintritt in den Wärmetauscher eine Base **B** zugegeben werden.

In Fig. 3 ist schematisch eine weitere Ausführungsform des erfindungsgemäßen Verfahrens zur Spaltung von CHP dargestellt. Über Leitung **a** wird ein Konzentrat, welches das zu spaltende CHP aufweist über einen Mischer **M** mit dem Spaltprodukt **c** aus dem Reaktor **Ra** zu einem Gemisch vermischt. Über Leitung **s** kann, bei Einsatz eines homogenen Katalysators, dieser in das Spaltprodukt **c** gegeben werden. Eine Teilmenge des Gemisches aus dem Mischer **M** wird in einen ersten Reaktor **Ra**, den Niedertemperaturspaltreaktor, eingespeist. Bei dem Niedertemperaturspaltreaktor **Ra** muss es sich nicht um nur einen Reaktor handeln, der z.B. als Rohrreaktor mit Rückführung oder als rückvermischter Apparat ausgcführt sein kann, es können auch mehrere hintereinander geschaltete Reaktoren als Niedertemperaturspaltreaktor bezeichnet werden. Das aus dem Niedertemperaturspaltreaktor austretende Niedertemperaturspaltprodukt wird im Falle eines Rohrreaktors über Leitung c in de Mischer **M** zurück gefahren. Über Leitung **b'** wird aus dem Mischer **M** eine Teilmenge des Gemisches in einen zweiten Spaltreaktor **Rb**, den Hochtemperaturspaltreaktor gefahren, in welchem die Hochtemperaturspaltung stattfindet. Vor dem Reaktor **Rb** kann über eine Leitung optional Wasser (**H**_{**2**}**O**) in die Teilmenge des Gemisches gefahren werden. Über Leitung **d** verlässt das thermisch gespaltene Hochtemperaturspaltprodukt den Reaktor **Rb** und kann einer Aufarbeitung zugeführt werden. Mittels des Wärmetauschers **W** kann dem Hochtemperaturspaltprodukt Wärme entzogen werden. Optional kann dem Hochtemperaturspaltprodukt vor Eintritt in den Wärmetauscher eine Base **B** zugegeben werden.

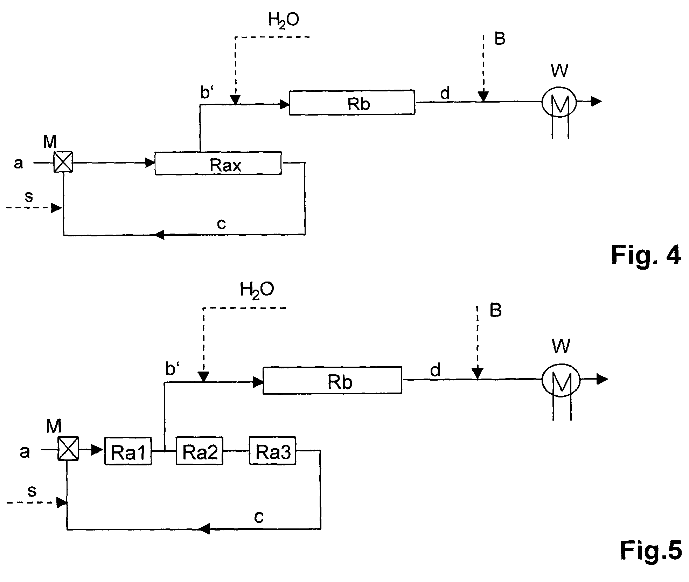

In Fig. 4 ist schematisch eine weitere Ausführungsform des erfindungsgemäßen Verfahrens zur Spaltung von CHP dargestellt. Über Leitung **a** wird ein Konzentrat, welches das zu spaltende CHP aufweist über einen Mischer **M** mit dem Spaltprodukt **c**, aus dem Reaktor **Rax** zu einem Gemisch vermischt. Über Leitung **s** kann, bei Einsatz eines homogenen Katalysators, dieser in das Spaltprodukt **c** gegeben werden. Eine Teilmenge des Gemisches aus dem Mischer **M** wird in einen ersten Reaktor **Rax**, den Niedertemperaturspaltreaktor, eingespeist, der in einen Bereich, in welchem die Vorspaltung stattfindet und einen Bereich in welchem die Niedertemperaturspaltung durchgeführt wird, aufgeteilt ist. Bei dem Niedertemperaturspaltreaktor **Rax** kann es sich z.B. um einen Rohrreaktor handeln. Das aus dem Niedertemperaturspaltreaktor austretende Niedertemperaturspaltprodukt wird im Falle eines Rohrreaktors über Leitung **c** in den Mischer **M** zurück gefahren. Über Leitung **b'** wird aus dem Niedertemperaturspaltreaktor **Rax** eine Teilmenge des Gemisches aus der Vorspaltung in einen zweiten Spaltreaktor **Rb**, den Hochtemperaturspaltreaktor gefahren, in welchem die Hochtemperaturspaltung stattfindet. Vor dem Reaktor **Rb** kann über eine Leitung optional Wasser (**H**_{**2**}**O**) in die Teilmenge des Gemisches, welches aus der Vorspaltung stammt, gefahren werden. Über Leitung **d** verlässt das thermisch gespaltene Hochtemperaturspaltprodukt den Reaktor **Rb** und kann einer Aufarbeitung zugeführt werden. Mittels des Wärmetauschers **W** kann dem Hochtemperaturspaltprodukt Wärme entzogen werden. Optional kann dem Hochtemperaturspaltprodukt vor Eintritt in den Wärmetauscher eine Base **B** zugegeben werden.

In Fig. 5 ist schematisch eine weitere Ausführungsform des erfindungsgemäßen Verfahrens zur Spaltung von CHP dargestellt. Über Leitung **a** wird ein Konzentrat, welches das zu spaltende CHP aufweist, über einen Mischer **M** mit dem Spaltprodukt **c**, aus dem Reaktor **Ra3** zu einem Gemisch vermischt. Über Leitung **s** kann, bei Einsatz eines homogenen Katalysators, dieser in das Spaltprodukt **c** gegeben werden. Das Gemisch aus dem Mischer **M** wird in einen ersten Vorspaltungsreaktor **Ra1** eingespeist. Das aus diesem Reaktor austretende Gemisch wird zum Teil in einen Niedertemperaturspaltreaktor **Ra2** und dieser wiederum in einen zweiten Niedertemperaturspaltreaktor **Ra3** überführt. Das aus dem Niedertemperaturspaltreaktor **Ra3** austretende Niedertemperaturspaltprodukt wird über Leitung **c** in den Mischer **M** zurückgefahren. Über Leitung **b'** wird eine Teilmenge des Gemisches aus der Vorspaltung, die aus dem Vorspaltungsreaktor **Ra1** austritt, in einen Hochtemperaturspaltreaktor **Rb** gefahren, in welchem die Hochtemperaturspaltung stattfindet. Vor dem Reaktor **Rb** kann über eine Leitung optional Wasser (**H**_{**2**}**O**) in die Teilmenge des Gemisches aus der Vorspaltung gefahren werden. Über Leitung **d** verlässt das Hochtemperaturspaltprodukt den Hochtemperaturspaltreaktor **Rb** und kann einer Aufarbeitung zugeführt werden. Mittels des Wärmetauschers **W** kann dem Hochtemperaturspaltprodukt Wärme entzogen werden. Optional kann dem Hochtemperaturspaltprodukt vor Eintritt in den Wärmetauscher eine Base **B** zugegeben werden.

### Beispiel 1: Gemäß dem Stand der Technik

In einer Spaltung gemäß Fig. 1 werden 20 t/h eines Konzentrates mit einem Gehalt an CHP von 68 Gew.-% eingespeist. Die Menge des als Kreislauf rückgeführten Spaltproduktstroms beträgt 210 t/h, dass heißt das Kreislaufverhältnis beträgt 10,5. Der Rohrreaktor ist so dimensioniert, dass bei einer Schwefelsäurekonzentration von 320 wppm, einem Wassergehalt von 0,7 Gew.-% und einer Reaktionstemperatur von 50 °C die CHP-Konzentration am Ausgang des Rohrreaktors 0,5 Gew.-% und die DCP-Konzentration 5,1 Gew.-% betragen. Um die anschließende thermische Nachbehandlung bei einer Temperatur von z. B. 115 °C betreiben zu können, muss die CHP-Konzentration im Spaltproduktstrom zur thermischen Nachbehandlung durch Einspeisung von Konzentrat von 0,5 auf 6,4 Gew.-% angehoben werden, das heißt, es müssen 1,9 t/h eines 68 Gew.-%-igen Konzentrates zudosiert werden.

### Beispiel 2: erfindungsgemäß

Eine Spaltung wird analog Beispiel 1 betrieben, jedoch wird eine Teilmenge des Gemisches aus Konzentrat und Spaltprodukt nicht am Ausgang des Rohrreaktors, sondern entsprechend Fig. 2 vor Eintritt in den als Kreislaufreaktor geschalteten Niedertemperaturspaltreaktor abgenommen und der Hochtemperaturspaltung zugeführt. Unter den in Beispiel 1 beschriebenen Bedingungen beträgt die CHP-Konzentration hier 6,4 %, so dass in der Hochtemperaturspaltung die gleiche maximale Temperatur von ca. 115 °C erreicht wird, ohne dass zusätzliches CHP-Konzentrat zudosiert werden muss.

## Patentansprüche

1. Verfahren zur säurekatalysierten Spaltung von Alkylarylhydroperoxiden, wobei ein Gemisch aus einem zumindest ein zu spaltendes Alkylarylhydroperoxid aufweisendem Konzentrat und einem Spaltprodukt, welches aus der Spaltung eines Alkylarylhydroperoxid erhalten wird, erzeugt wird, dieses Gemisch in zumindest zwei Teilmengen aufgetrennt wird und die Alkylarylhydroperoxide in diesen Teilmengen parallel bei unterschiedlichen Temperaturen gespalten werden.

2. Verfahren nach Anspruch 1,
**dadurch gekennzeichnet,**
**dass** die Konzentrationen an Alkylarylhydroperoxiden in den Teilmengen des Gemisches direkt nach dem Auftrennen des Gemisches gleich sind.

3. Verfahren nach Anspruch 1 oder 2,
**dadurch gekennzeichnet,**
**dass** zumindest eines der Alkylarylhydroperoxide Cumolhydroperoxid ist.

4. Verfahren nach zumindest einem der Ansprüche 2 oder 3,
**dadurch gekennzeichnet,**
**dass** die Teilmengen des Gemisches direkt nach dem Auftrennen des Gemisches Cumolhydroperoxid in einer Konzentration von bis zu 20 Gew.-% aufweist.

5. Verfahren nach Anspruch 4,
**dadurch gekennzeichnet,**
**dass** die Teilmengen des Gemisches direkt nach dem Auftrennen des Gemisches jeweils Cumolhydroperoxid in einer Konzentration von 10 bis 15 Gew.-% aufweisen.

6. Verfahren nach einem der Ansprüche 1 bis 5,
**dadurch gekennzeichnet,**
**dass** das Auftrennen des Gemisches in zumindest zwei Teilmengen während der Herstellung des Gemisches erfolgt.

7. Verfahren nach einem der Ansprüche 1 bis 5,
**dadurch gekennzeichnet,**
**dass** das Auftrennen des Gemisches in zumindest zwei Teilmengen nach der Herstellung des Gemisches aber vor einer ersten Behandlung des Gemisches zur Spaltung von zumindest einem Alkylarylhydroperoxid in einem Reaktor erfolgt.

8. Verfahren nach einem der Ansprüche 1 bis 5,
**dadurch gekennzeichnet,**
**dass** das Auftrennen des Gemisches in zumindest zwei Teilmengen nach der Herstellung des Gemisches und während, aber zumindest vor Beendigung, zumindest einer Behandlung des Gemisches zur Spaltung von zumindest einem Alkylarylhydroperoxid in zumindest einem Reaktor erfolgt.

9. Verfahren nach Anspruch 8,
**dadurch gekennzeichnet,**
**dass** das Gemisch zur Spaltung zumindest eines Alkylarylhydroperoxids in zumindest einem Reaktor bei 45 bis 75 °C behandelt wird.

10. Verfahren nach Anspruch 9,
**dadurch gekennzeichnet,**
**dass** die Behandlung in zumindest einem Reaktor, der mit einer Vorrichtung zur Abfuhr von Wärmeenergie ausgestattet ist, durchgeführt wird.

11. Verfahren nach zumindest einem der Ansprüche 1 bis 5,
**dadurch gekennzeichnet,**
**dass** das Auftrennen des Gemisches in zumindest zwei Teilmengen des Gemisches nach einer Zeit von 0,01 bis 600 Sekunden nach Herstellung des Gemisches erfolgt.

12. Verfahren nach zumindest einem der Ansprüche 1 bis 11,
**dadurch gekennzeichnet,**
**dass** das Gemisch aus zumindest ein zu spaltendes Alkylarylhydroperoxid aufweisendem Konzentrat und einem Spaltprodukt durch Mischen des Konzentrats mit dem Spaltprodukt im Verhältnis von 1:100 bis 1:1 hergestellt wird.

13. Verfahren nach Anspruch 12,
**dadurch gekennzeichnet,**
**dass** das Gemisch aus zumindest ein zu spaltendes Alkylarylhydroperoxid aufweisendem Konzentrat und einem Spaltprodukt durch Mischen des Konzentrats mit dem Spaltprodukt im Verhältnis von 1:50 bis 1:5 hergestellt wird.

14. Verfahren gemäß zumindest einem der Ansprüche 1 bis 13.
**dadurch gekennzeichnet**
**dass** zumindest eine der abgetrennten Teilmengen des Gemisches zur Spaltung zumindest eines Alkylarylhydroperoxides bei einer Temperatur von 45 bis 90 °C behandelt wird.

15. Verfahren nach Anspruch 14,
**dadurch gekennzeichnet**
**dass** die abgetrennte Teilmenge des Gemisches bei einer Temperatur von 45 bis 75 °C behandelt wird.

16. Verfahren nach zumindest einem der Ansprüche 14 bis 15,
**dadurch gekennzeichnet,**
**dass** die Behandlung in zumindest zwei Spaltreaktoren durchgeführt wird.

17. Verfahren nach Anspruch 16,
**dadurch gekennzeichnet,**
**dass** die Behandlung in zumindest drei Spaltreaktoren durchgeführt wird.

18. Verfahren nach Anspruch 16 oder 17,
**dadurch gekennzeichnet,**
**dass** die Spaltreaktoren in Serie geschaltet sind und zumindest eine Vorrichtung zum Abführen von Wärmeenergie aufweisen.

19. Verfahren nach Anspruch 16 bis 18,
**dadurch gekennzeichnet,**
**dass** die Temperatur in zumindest zwei der Spaltreaktoren einen Temperaturunterschied von 0 bis 10 °C aufweisen.

20. Verfahren nach zumindest einem der Ansprüche 1 bis 19,
**dadurch gekennzeichnet,**
**dass** zumindest eine Teilmenge des Gemisches bei einer Temperatur von über 100 °C behandelt wird.

21. Verfahren nach Anspruch 20,
**dadurch gekennzeichnet,**
**dass** diese Teilmenge des Gemisches bei einer Temperatur von über 130 °C behandelt wird.

22. Verfahren nach Anspruch 20 oder 21,
**dadurch gekennzeichnet,**
**dass** diese Teilmenge des Gemisches in einem Spaltreaktor durch eine in diesem Spaltreaktor ablaufenden exothermen Reaktion auf eine Temperatur von über 100 °C erwärmt wird.

23. Verfahren nach Anspruch 22,
**dadurch gekennzeichnet,**
**dass** eine der in diesem Spaltreaktor ablaufenden exothermen Reaktionen die Spaltung eines Alkylarylhydroperoxids ist.

24. Verfahren nach Anspruch 23,
**dadurch gekennzeichnet,**
**dass** die exotherme Reaktion die Spaltung von Cumolhydroperoxid ist.

25. Verfahren nach zumindest einem der Ansprüche 20 bis 24,
**dadurch gekennzeichnet,**
**dass** der Teilmenge, die bei einer Temperatur über 100 °C behandelt wird, Wasser zudosiert wird.

26. Verfahren nach zumindest einem der Ansprüche 20 bis 25.
**dadurch gekennzeichnet,**
**dass** das Spaltprodukt der Teilmenge, die bei einer Temperatur über 100 °C behandelt wurde, nach der Behandlung abgekühlt wird.

27. Verfahren nach Anspruch 26,
**dadurch gekennzeichnet,**
**dass** das Spaltprodukt der Teilmenge, die bei einer Temperatur über 100 °C behandelt wurde, vor dem Abkühlen neutralisiert wird.

28. Verfahren nach Anspruch 27,
**dadurch gekennzeichnet,**
**dass** das Spaltprodukt der Teilmenge, die bei einer Temperatur über 100 °C behandelt wurde, mit einer Base, ausgewählt aus wässrigen oder organischen Lösungen von NaOH oder Phenolatlauge, neutralisiert wird.

29. Verfahren nach zumindest einem der Ansprüche 1 bis 28,
**dadurch gekennzeichnet,**
**dass** als Spaltprodukt, welches aus der Spaltung eines Alkylarylhydroperoxid erhalten wird und welches zum Erzeugen des Gemisches aus Spaltprodukt und Konzentrat verwendet wird, das Spaltprodukt aus der Behandlung zumindest einer der Teilmengen des Gemisches eingesetzt wird.

30. Verfahren nach Anspruch 29,
**dadurch gekennzeichnet,**
**dass** als Spaltprodukt das Spaltprodukt einer Teilmenge des Gemisches, welche bei einer Temperatur von 45 bis 90 °C behandelt wurde, eingesetzt wird.

31. Verfahren nach einem der Ansprüche 1-30, wobei das Alkylarylhydroperoxid Cumolhydroperoxid ist und Phenol hergestellt wird.

## Claims

1. A process for the acid-catalyzed cleavage of alkylaryl hydroperoxides, which comprises producing a mixture of a concentrate comprising at least one alkylaryl hydroperoxide to be cleaved and a cleavage product obtained from the cleavage of an alkylaryl hydroperoxide, dividing this mixture into at least two parts, and cleaving the alkylaryl hydroperoxides in these parts in parallel at different temperatures.

2. The process as claimed in claim 1, wherein the concentrations of alkylaryl hydroperoxides in the parts of the mixture immediately after division of the mixture are identical.

3. The process as claimed in claim 1 or 2, wherein at least one of the alkylaryl hydroperoxides is cumene hydroperoxide.

4. The process as claimed in at least one of claims 2 to 3, wherein the parts of the mixture immediately after division of the mixture comprise cumene hydroperoxide in a concentration of up to 20 percent by weight.

5. The process as claimed in claim 4, wherein the parts of the mixture immediately after division of the mixture each comprise cumene hydroperoxide in a concentration of from 10 to 15 percent by weight.

6. The process as claimed in any of claims 1 to 5, wherein the division of the mixture into at least two parts is carried out during production of the mixture.

7. The process as claimed in any of claims 1 to 5, wherein the division of the mixture into at least two parts is carried out after production of the mixture but before a first treatment of the mixture for the cleavage of at least one alkylaryl hydroperoxide in a reactor.

8. The process as claimed in any of claims 1 to 5, wherein the division of the mixture into at least two parts is carried out after production of the mixture and during, but at least before completion of, at least one treatment of the mixture for the cleavage of at least one alkylaryl hydroperoxide in at least one reactor.

9. The process as claimed in claim 8, wherein the mixture for the cleavage of at least one alkylaryl hydroperoxide is treated in at least one reactor at from 45 to 75°C.

10. The process as claimed in claim 9, wherein the treatment is carried out in at least one reactor which is equipped with a facility for removing heat energy.

11. The process as claimed in at least one of claims 1 to 5, wherein the division of the mixture into at least two parts of the mixture is carried out after a time of from 0.01 to 600 seconds after production of the mixture.

12. The process as claimed in at least one of claims 1 to 11, wherein the mixture of a concentrate comprising at least one alkylaryl hydroperoxide to be cleaved and a cleavage product is produced by mixing the concentrate with the cleavage product in a ratio of from 1:100 to 1:1.

13. The process as claimed in claim 12, wherein the mixture of a concentrate comprising at least one alkylaryl hydroperoxide to be cleaved and a cleavage product is produced by mixing the concentrate with the cleavage product in a ratio of from 1:50 to 1:5.

14. The process as claimed in at least one of claims 1 to 13. wherein at least one of the separated-off parts of the mixture is treated at a temperature of from 45 to 90°C for the cleavage of at least one alkylaryl hydroperoxide.

15. The process as claimed in claim 14, wherein the separated-off part of the mixture is treated at a temperature of from 45 to 75°C.

16. The process as claimed in at least one of claims 14 to 15, wherein the treatment is carried out in at least two cleavage reactors.

17. The process as claimed in claim 16, wherein the treatment is carried out in at least three cleavage reactors.

18. The process as claimed in claim 16 or 17, wherein the cleavage reactors are connected in series and have at least one facility for removing heat energy.

19. The process as claimed in claim 16 to 18, wherein the temperatures in at least two of the cleavage reactors have a temperature difference of from 0 to 10°C.

20. The process as claimed in at least one of claims 1 to 19, wherein at least one part of the mixture is treated at a temperature above 100°C.

21. The process as claimed in claim 20, wherein this part of the mixture is treated at a temperature above 130°C.

22. The process as claimed in claim 20 or 21, wherein this part of the mixture is heated to a temperature above 100°C in a cleavage reactor by means of an exothermic reaction occurring in this cleavage reactor.

23. The process as claimed in claim 22, wherein one of the exothermic reactions occurring in this cleavage reactor is the cleavage of an alkylaryl hydroperoxide.

24. The process as claimed in claim 23, wherein the exothermic reaction is the cleavage of cumene hydroperoxide.

25. The process as claimed in at least one of claims 20 to 24, wherein water is added to the part of the mixture which is treated at a temperature above 100°C.

26. The process as claimed in at least one of claims 20 to 25, wherein the cleavage product of the part of the mixture which was treated at a temperature above 100°C is cooled after the treatment.

27. The process as claimed in claim 26, wherein the cleavage product of the part of the mixture which was treated at a temperature above 100°C is neutralized prior to cooling.

28. The process as claimed in claim 27, wherein the cleavage product of the part of the mixture which was treated at a temperature above 100°C is neutralized by means of a base selected from among aqueous or organic solutions of Na0H or sodium phenoxide.

29. The process as claimed in any of claims 1 to 28, wherein the cleavage product which is obtained from the cleavage of an alkylaryl hydroperoxide and which is used for producing the mixture of cleavage product and concentrate is the cleavage product from the treatment of at least one of the parts of the mixture.

30. The process as claimed in claim 29, wherein the cleavage product used is the cleavage product of a part of the mixture which has been treated at a temperature of from 45 to 90°C.

31. The process as claimed in any of claims 1 to 30, wherein the alkylaryl hydroperoxide is cumene hydroperoxide and the product obtained is phenol.

## Revendications

1. Procédé pour la scission à catalyse acide d'hydroperoxydes d'alkylaryle, dans lequel on produit un mélange constitué d'un concentré présentant au moins un hydroperoxyde d'alkylaryle à scinder et d'un produit de scission, provenant de la scission d'un hydroperoxyde d'alkylaryle, ce mélange est séparé en au moins deux parties et les hydroperoxydes d'alkylaryle sont scindés en parallèle dans des parties à des températures différentes.

2. Procédé selon la revendication 1, **caractérisé en ce que** les concentrations en hydroperoxydes d'alkylaryle dans les parties du mélange sont identiques immédiatement après la séparation du mélange.

3. Procédé selon la revendication 1 ou 2, **caractérisé en ce qu'**au moins l'un des hydroperoxydes d'alkylaryle est de l'hydroperoxyde de cumol.

4. Procédé selon l'une des revendications 2 ou 3, **caractérisé en ce que** les parties du mélange présentent, immédiatement après la séparation du mélange, l'hydroperoxyde de cumol en une concentration jusqu'à 20% en poids.

5. Procédé selon la revendication 4, **caractérisé en ce que** les parties du mélange présentent, immédiatement après la séparation du mélange, à chaque fois de l'hydroperoxyde de cumol en une concentration de 10 à 15% en poids.

6. Procédé selon l'une des revendications 1 à 5, **caractérisé en ce que** la séparation du mélange se fait en au moins deux parties pendant la production du mélange.

7. Procédé selon l'une des revendications 1 à 5, **caractérisé en ce que** la séparation du mélange se fait en au moins deux parties après la production du mélange, mais avant un premier traitement du mélange pour la scission d'au moins un hydroperoxyde d'alkylaryle dans un réacteur.

8. Procédé selon l'une des revendications 1 à 5, **caractérisé en ce que** la séparation du mélange se fait en au moins deux parties après la production du mélange, mais au moins avant la fin d'au moins un traitement du mélange pour la scission d'au moins un hydroperoxyde d'alkylaryle dans au moins un réacteur.

9. Procédé selon la revendication 8, **caractérisé en ce que** le mélange est traité pour la scission d'au moins un hydroperoxyde d'alkylaryle dans au moins un réacteur à une température de 45 à 75°C.

10. Procédé selon la revendication 9, **caractérisé en ce que** le traitement est entrepris dans au moins un réacteur équipé d'un dispositif pour la dissipation d'énergie thermique.

11. Procédé selon l'une au moins des revendications 1 à 5, **caractérisé en ce que** la séparation du mélange en au moins deux parties du mélange est entreprise après une durée de 0,01 à 600 secondes après la production du mélange.

12. Procédé selon l'une au moins des revendications 1 à 11, **caractérisé en ce que** le mélange constitué d'un concentré présentant au moins un hydroperoxyde d'alkylaryle et d'un produit de scission est préparé par mélange du concentré au produit de scission en une proportion de 1 : 100 à 1 : 1.

13. Procédé selon la revendication 12, **caractérisé en ce que** le mélange constitué d'un concentré présentant au moins un hydroperoxyde d'alkylaryle et d'un produit de scission est préparé par mélange du concentré au produit de scission en une proportion de 1 : 50 à 1: 5.

14. Procédé selon l'une au moins des revendications 1 à 13, **caractérisé en ce qu'**au moins l'une des parties séparées du mélange est traitée pour la scission d'au moins un hydroperoxyde d'alkylaryle à une température de 45 à 90°C.

15. Procédé selon le revendication 14, **caractérisé en ce que** la quantité partielle séparée du mélange est traitée à une température de 45 à 75°C.

16. procédé selon l'une au moins des revendications 14 à 15, **caractérisé en ce que** le traitement est entrepris dans au moins deux réacteurs de scission.

17. Procédé selon la revendication 16, **caractérisé en ce que** le traitement est entrepris dans au moins trois réacteurs de scission.

18. Procédé selon la revendication 16 ou 17, **caractérisé en ce que** les réacteurs de scission sont reliés en série et présentent au moins un dispositif pour la dissipation d'énergie thermique.

19. Procédé selon les revendications 16 à 18, **caractérisé en ce que** les températures dans au moins deux des réacteurs de scission présentent une différence de température de O à 10°C.

20. Procédé selon l'une des revendications 1 à 19, **caractérisé en ce qu'**au moins une partie du mélange est traitée à une température de plus de 100°C.

21. Procédé selon la revendication 20, **caractérisé en ce que** cette partie du mélange est traitée à une température de plus de 130°C.

22. Procédé selon la revendication 20 ou 21, **caractérisé en ce que** cette partie du mélange est chauffée, dans un réacteur de scission, à une température de plus de 100°C par une réaction exothermique se déroulant dans ce réacteur de scission.

23. Procédé selon la revendication 22, **caractérisé en ce que** l'une des réactions exothermiques se déroulant dans ce réacteur de scission est la scission d'un hydroperoxyde d'alkylaryle.

24. Procédé selon la revendication 23, **caractérisé en ce que** la réaction exothermique est la scission d'hydroperoxyde de cumol.

25. Procédé selon l'une au moins des revendication 20 à 24, **caractérisé en ce que** l'on ajoute de l'eau à la partie qui est traitée à une température de plus de 100°C.

26. Procédé selon l'une au moins des revendications 20 à 25, **caractérisé en ce que** le produit de scission de la partie qui a été traitée à une température de plus de 100°C est refroidie après le traitement.

27. Procédé selon la revendication 26, **caractérisé en ce que** le produit de scission de la partie qui a été traitée à une température de plus de 100°C est neutralisé avant le refroidissement.

28. Procédé selon la revendication 27, **caractérisé en ce que** le produit de scission de la partie qui a été traitée à une température de plus de 100°C est neutralisé par une base choisie parmi des solutions aqueuses ou organiques de NaOH ou de lessive de phénolate.

29. Procédé selon l'une au moins des revendications 1 à 28, **caractérisé en ce que** l'on met en oeuvre, comme produit de scission obtenu par la scission d'un hydroperoxyde d'alkylaryle et qui est utilisé pour la production du mélange de produit de scission et de concentré, le produit de scission provenant du traitement d'au moins l'une des parties, du mélange.

30. Procédé selon la revendication 29, **caractérisé en ce que** l'on met en oeuvre comme produit de scission le produit de scission d'une partie du mélange, qui a été traitée à une température de 45 à 90°C.

31. Procédé selon l'une des revendications 1 à 30, dans lequel l'hydroperoxyde d'alkylaryle est de l'hydroperoxyde de cumol et que l'on prépare du phénol.
